# EUROPEAN PATENT APPLICATION

(11) **EP 3 508 236 A1**
(43) Date of publication of application: **10.07.2019**
(21) Application number: 18150449.9
(22) Date of filing: 05.01.2018
(51) Int. Cl.: A61M 5/20, A61M 5/145

(54) **AN INJECTION DEVICE WITH A DRIVER MECHANISM WITH A SPRING AND A CABLE**

(71) Applicant: Medicom Innovation Partner a/s, 7600 Struer (DK)
(72) Inventor: OLESEN, Jan, 7500 Holstebro (DK); BECHMANN, Søren, 7500 Holstebro (DK); CHRISTENSEN, Andreas Hou, 7600 Struer (DK); RAHBEK, Niels Skovby, 7500 Holstebro (DK); DAUL, Lucile, 7600 Struer (DK)
(74) Representative: Inspicos P/S

(57) **Abstract**

An injection device comprising a housing, a syringe arranged inside the housing and a driver mechanism is disclosed. A movable plunger is arranged inside the syringe, and the driver mechanism drives the movable plunger. The driver mechanism comprises a spring and a cable operatively connected to the movable plunger of the syringe in such a manner that the movable plunger can be moved in a first direction by means of a spring force applied by the spring, and the movable plunger can be moved in a second direction being opposite to the first direction by means of a pulling force applied via the cable.

## Description

### FIELD OF THE INVENTION

The present invention relates to an injection device comprising a syringe with a movable plunger and a driver mechanism for driving the movable plunger of the syringe.

### BACKGROUND OF THE INVENTION

Injection devices are used for injecting medical drugs in liquid form subcutaneously or intravenously into patients. Some kinds of medical drugs are normally administered by professional health staff, often at a hospital or medical clinic. This is, e.g., the case when the medical drug is a complex drug or if administration of the medical drug is complicated and/or if there is a high risk of errors occurring during administration of the drug.

The need for going to a hospital or a medical clinic in order to receive relevant medical drugs, and the dependence on the availability of professional medical staff for administering the medical drug is cumbersome for patients, and it requires planning and introduces waiting time. It would therefore be an improvement of the quality of life for patients being dependent on such medical drugs to be able to self-administer the medical drug, even in the case of large volume injections.

US 3,797,488 discloses an ampoule applicator comprising a hollow, cylindrical body. The applicator includes a spring-biased force-applying member for actuating the ampoule. The force-applying member may be retracted against the bias of a spring by a cocking lever which rotates a pulley at one end of the applicator. A flexible cable is fixed at one end to the force-applying member and is fixed at its other end to the pulley so that, as the pulley is rotated, the cable is wrapped about the pulley and the force-applying member is retracted. The force-applying member is released by inserting a pin in a slot to move a ball away from an ampoule-mounting end of the applicator.

### DESCRIPTION OF THE INVENTION

It is an object of embodiments of the invention to provide an injection device which allows accurate self-administration of medical drug.

It is a further object of embodiments of the invention to provide an injection device which is wearable and/or compact while supporting large volume injections.

The invention provides an injection device comprising:
- a housing,
- a syringe arranged inside the housing, the syringe having a movable plunger arranged therein,
- a driver mechanism arranged inside the housing for driving the movable plunger of the syringe,

wherein the driver mechanism comprises a spring and a cable operatively connected to the movable plunger of the syringe in such a manner that the movable plunger can be moved in a first direction by means of a spring force applied by the spring, and the movable plunger can be moved in a second direction being opposite to the first direction by means of a pulling force applied via the cable.

Thus, the invention provides an injection device. In the present context the term 'injection device' should be interpreted to mean a device which is used for injecting medical drug subcutaneously or intravenously into a patient.

The injection device according to the invention comprises a housing and a syringe arranged inside the housing. In the present context the term 'housing' should be interpreted to mean a substantially closed object defining an outer boundary, e.g. in the form of one or more walls, and an interior part enclosed by the outer boundary. The syringe is, thus arranged in the interior part of the housing.

The syringe comprises a movable plunger arranged therein. Thereby medical drug contained in the syringe can be expelled from the syringe by moving the plunger inside the syringe along a first direction. Furthermore, medical drug, diluent or other suitable substances may be retrieved into the syringe by moving the plunger inside the syringe along a second, reverse direction.

The injection device further comprises a driver mechanism arranged inside the housing for driving the movable plunger of the syringe. Accordingly, the movements of the plunger described above are controlled by means of the driver mechanism.

The driver mechanism comprises a spring and a cable operatively connected to the movable plunger of the syringe in such a manner that the movable plunger can be moved in a first direction by means of a spring force applied by the spring, and the movable plunger can be moved in a second direction being opposite to the first direction by means of a pulling force applied via the cable.

Thus, the driver mechanism is capable of moving the plunger in a direction which causes medical drug to be expelled from the syringe, as well as in a direction which causes medical drug, diluent, or other suitable substances to be retrieved into the syringe.

The movement in the first direction, caused by the spring force of the spring, preferably causes medical drug to be expelled from the syringe, and the movement in the second direction, caused by the pulling force applied via the cable, preferably causes medical drug, diluent or other suitable substances to be retrieved into the syringe. In this case medical drug can easily be expelled from the syringe, simply by releasing mechanical energy previously stored in the spring.

Furthermore, medical drug, diluent or other suitable substances can easily be retrieved into the syringe by applying a suitable pulling force to the plunger via the cable.

The combination of the driver mechanism being capable of controlling movement of the plunger along the first direction by means of the spring, and of controlling movement of the plunger along the second direction by means of the cable allows the driver mechanism to control the position of the plunger inside the syringe very accurately. Thereby drug contained in the syringe can be very accurately administered. This allows even complex drug administration to be performed as self-administration, using an injection device according to the invention.

Furthermore, applying the pulling force by means of a cable allows the injection device to be designed with a compact shape and size, e.g. allowing it to be wearable, while supporting large volume injections.

The spring may be a compressible spring, and movement of the movable plunger in the second direction by means of the pulling force applied via the cable may further cause the spring to be compressed.

According to this embodiment, mechanical energy is automatically stored in the spring when the plunger is moved in the second direction, and thereby the mechanical energy is immediately ready to be released in order to move the plunger along the first direction as soon as the plunger has been moved a sufficient distance along the second direction. For instance, medical drug may initially be retrieved into the syringe, e.g. from a vial, by applying a pulling force to the plunger by means of the cable, while simultaneously compressing the spring and storing mechanical energy therein. When a sufficient amount of medical drug has been retrieved into the syringe in this manner, an injection needle may be connected to the syringe and inserted into a relevant injection position. Then the mechanical energy stored in the spring may be released, thereby causing the plunger to move along the first direction, due to the spring force acting on the stopper, thereby causing the medical drug to be expelled from the syringe.

As an alternative, the spring may be any other suitable kind of spring and/or mechanical energy may be stored in the spring in any other suitable manner, independently of the movement of the plunger along the second direction.

The cable may further be arranged to control a velocity of movement of the plunger when the plunger is moved in the first direction by means of the spring force. According to this embodiment, instead of applying the full spring force to the plunger when it is desired to move the plunger in the first direction, the plunger may be 'held back' by means of the cable. Accordingly, the velocity of the plunger movement along the first direction, and thereby the dose as well as the speed at which the medical drug is expelled, is accurately controlled.

For instance, one end of the spring may be rigidly mounted, while another end of the spring may be connected to the cable. The cable may extend along the spring, e.g. through a centre part thereof. Thereby the cable will apply a pulling force to the spring which acts in a direction being opposite to the direction in which the spring force acts, and thereby the cable controls the expansion of the spring.

The driver mechanism may further comprise a motor arranged to provide the pulling force via the cable. According to this embodiment the movement of the plunger along the second direction is provided by means of the motor. This allows the movement along the second direction to be controlled in a very accurate manner. The motor may advantageously be an electric motor.

For instance, the motor may be connected to the cable via a reel transforming a rotational torque of the motor into a translational pulling force in the cable.

The injection device may further comprise a coupling mechanism for coupling the motor and the cable. This could, e.g., include a reel as described above.

The coupling mechanism may comprise a gear mechanism. According to this embodiment, the movements of the motor are not transferred one-to-one to the cable. Instead the gearing of the gear mechanism may provide very small movements of the cable, thereby allowing the cable, and thereby the plunger, to be moved in a very accurate manner.

The gear mechanism may, e.g., include a worm gear. Alternatively or additionally, the gear mechanism may include one or more gear stages.

Alternatively or additionally, the coupling mechanism may comprise a spline connection. According to this embodiment, force is transferred from the motor to the cable by means of the spline connection. This makes it easy to decouple the motor from the cable, since this can be done simply be decoupling the spline connection. This could, e.g., be relevant if the motor forms part of a reusable sub-system of the injection device and the cable forms part of a disposable sub-system of the injection device. This will be described in further detail below.

The spline connection may, e.g., comprise a spline shaft being connected to the motor, e.g. via a gear mechanism, and a female spline part being connected to the cable, e.g. via a pulley or a wheel onto which the cable may be rolled. As an alternative, a female spline connection may be connected to the motor and a spline shaft may be connected to the cable. Such spline connections allow rotational movements to be transferred between the motor and the pulley or wheel. The rotation of the pulley or wheel determines a length of the cable being rolled out, and thereby the rotational movement of the pulley or wheel is transformed into a translational movement of the cable.

The driver mechanism may further comprise one or more pulleys arranged to guide the cable. The pulley(s) allow the cable to change direction within the injection device. Thereby the mechanism used for providing the pulling force on the plunger can be made in a compact manner, and consequently a compact design of the injection device can be obtained.

For instance, the cable may have one end attached to a spring support on the plunger and an opposite end attached to a pulley or wheel having a part of the cable rolled thereon, as described above. One or more intermediate pulleys may be arranged along a path of the cable between the spring support and the pulley or wheel, the intermediate pulley(s) each causing a change in direction of the cable.

The injection device may further comprise position monitoring means for monitoring a position of the movable plunger. According to this embodiment, the position of the movable plunger can be accurately established at all times during operation of the injection device, during movement in the first direction due to the spring force, as well as during movement in the second direction due to the pulling force of the cable. Thereby an even more accurate control of the injection device is possible. Furthermore, it may thereby be determined whether or not the plunger is in the expected position, and thereby whether or not the plunger has moved as expected. In the case that it is determined that the plunger is not in an expected position, this may be due to movement of the plunger being inhibited, e.g. because the plunger has reached the end of the syringe or because the syringe is occluded.

The position monitoring means may comprise an optical sensor. In the case that the driver mechanism comprises a motor, the optical sensor may, e.g., be arranged to optically monitor rotation of a motor axle of the motor. Knowing how the motor and the plunger are connected, the movement of the plunger may then be derived from the rotation of the motor axle.

As an alternative, the position monitoring means may comprise a movably mounted magnet and a magnetic Hall sensor configured to detect a proximity of the movably mounted magnet. For instance, this may be implemented in the following manner. The magnet may be mounted in a movable part being biased in a direction towards the magnetic Hall sensor, e.g. by means of a spring. The cable may further be connected to the movable part in such a manner that when the movements of the plunger follows movements of, e.g. a motor and the cable is thereby tightened, the cable pulls the movable part in a direction away from the magnetic Hall sensor against the biasing force. In the case that the plunger is not following the movements of the motor, e.g. due to occlusion of the syringe, the cable is no longer tight, and the biasing force pulls the movable part towards the magnetic Hall sensor. Thus, measurements of the magnetic Hall sensor indicates whether or not the cable is tight, and thereby whether or not the plunger is moving as expected.

Alternatively, the position of the movable part may be detected in another manner, e.g. through using an inductive proximity sensor system, a capacitive proximity sensor system or even an optical proximity sensor system, etc.

By applying an infrared (IR) optical proximity sensor, the IR proximity sensor would be directly measuring the proximity of the movable part. This could for instance be implemented by having a visible pathway connecting a reusable part, where the IR sensor and light source are located, into a disposable part, where the moveable part is located. Compared, e.g., to a magnetic proximity sensor system, with an IR proximity sensor detection system, the cost on the disposable part would be further limited as no magnet would be needed.

As another alternative, the position monitoring means may form part of a motor arranged to provide the pulling force via the cable. For instance, the position monitoring means may be arranged to monitor motor input current of the motor. This could be used for monitoring the position of the movable plunger in the following manner.

When the movable plunger is moving as expected, the motor only has to roll out cable while the spring force is moving the movable plunger along the first direction. This requires a very small motor input current. However, when the movable plunger is moved in the second direction by means of the pulling force applied via the cable, a significantly larger motor input current is required, since the pulling must be sufficient to overcome stiction between the movable plunger and the inner wall of the syringe and possibly a spring compression force of the spring. Thus, when the movable plunger is moving in the manner which it is supposed to, a significant difference in motor input current is expected between moving the motor in one direction, causing rolling out of the cable while the movable plunger is moved by means of the spring force, and moving the motor in an opposite direction, causing the movable plunger to be pulled along the second direction by means of the cable.

When the movable plunger is unintentionally stopped during movement along the first direction, e.g. due to an occlusion, cable will be rolled out by the motor, but the cable will not be kept tight by the movable plunger. Accordingly, if the movement of the motor is subsequently reversed in order to apply a pulling force to the movable plunger, the loose cable is initially rolled up, but no pulling force is applied to the movable plunger, and no stiction or spring force needs to be overcome. Therefore the motor input current is, in this case, comparable to the motor input current during movement of the movable plunger along the first direction, due to the spring force, i.e. the motor input current is significantly smaller than expected.

A method for detecting occlusion could therefore be to reverse to movement of the motor for a brief period of time during movement of the movable plunger along the first direction, and measure the motor input current during the reverse movement. The measured motor input current may then be compared to a predefined threshold level. If the measured motor input current is above the threshold level, it can be concluded that the reverse movement of the motor actually results in a pulling force being applied to the movable plunger, and thereby that the movable plunger has so far moved as it is supposed to. However, if the measured motor input current is below the threshold level, it can be concluded that the reverse movement of the motor is not resulting in a pulling force being applied to the movable plunger, and thereby that the movable plunger has stopped its movement along the first direction. Accordingly, an occlusion has most likely occurred. The threshold level could be a fixed value, or it could be a relative value, such as twice the motor input current when rolling out the cable.

The injection device may further comprise a locking mechanism being configured to be in a locked position in which the spring is prevented from applying a spring force to the movable plunger and in an unlocked position in which the spring is allowed to apply a spring force to the movable plunger. According to this embodiment, mechanical energy may be stored in the spring. The stored mechanical energy may then be released in a controlled manner and at a selected point in time, thereby allowing the spring force to move the plunger in the first direction.

The locking mechanism may, e.g., comprise a locking pin, e.g. locking a member arranged to keep a tension on the spring. When the locking pin is removed, the member is no longer prevented from moving, and the mechanical energy stored in the spring is released.

As an alternative, the locking mechanism may comprise a lock on one or more pulleys arranged to guide the cable. When the pulley is locked, the cable is not able to move along the pulley, and thereby the cable may retain the spring. When the lock is removed, the cable is allowed to move, and thereby the mechanical energy stored in the spring is released.

The driver mechanism may form part of a disposable sub-system being removable from the housing. The disposable sub-system may further comprise the syringe. Thereby the parts of the injection device which are in contact with the medical drug can be removed from the housing and disposed of after use, while more expensive parts of the injection device, such as a motor, may be reused by arranging a new disposable sub-system in the housing.

The driver mechanism may further be configured to move the syringe. This could, e.g., be used during mounting of an injection needle on the syringe.

### BRIEF DESCRIPTION OF THE DRAWINGS

The invention will now be described in further detail with reference to the accompanying drawings in which
Figs. 1-3 are perspective views of an injection device according to an embodiment of the invention,
Figs. 4-6 show a reusable sub-system of the injection device of Figs. 1-3,
Figs. 7-10 show a disposable sub-system of the injection device of Figs. 1-3,
Figs. 11 and 12 show a locking mechanism for an injection device according to an embodiment of the invention,
Fig. 13 shows an injection device according to an embodiment of the invention, comprising a pre-mounted syringe,
Fig. 14 shows an injection device according to an embodiment of the invention, comprising a user inserted syringe,
Figs. 15-17 illustrate operation of an injection device according to an embodiment of the invention, and
Figs. 18 and 19 show an occlusion detection mechanism for an injection device according to an embodiment of the invention.

### DETAILED DESCRIPTION OF THE DRAWINGS

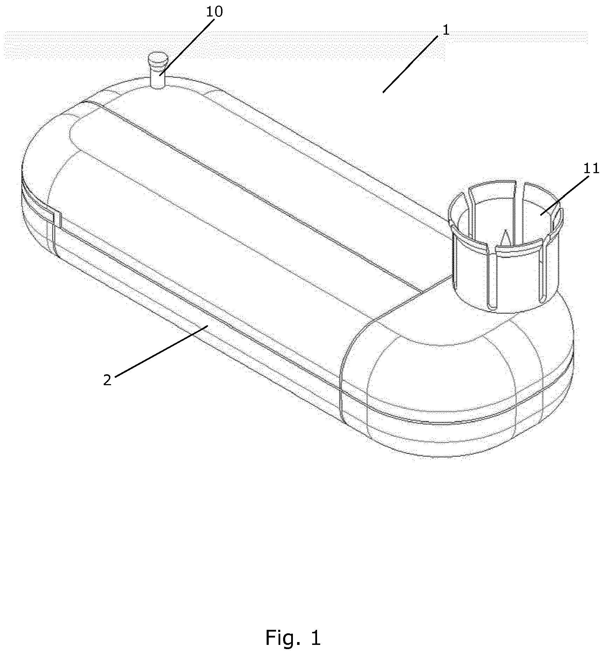

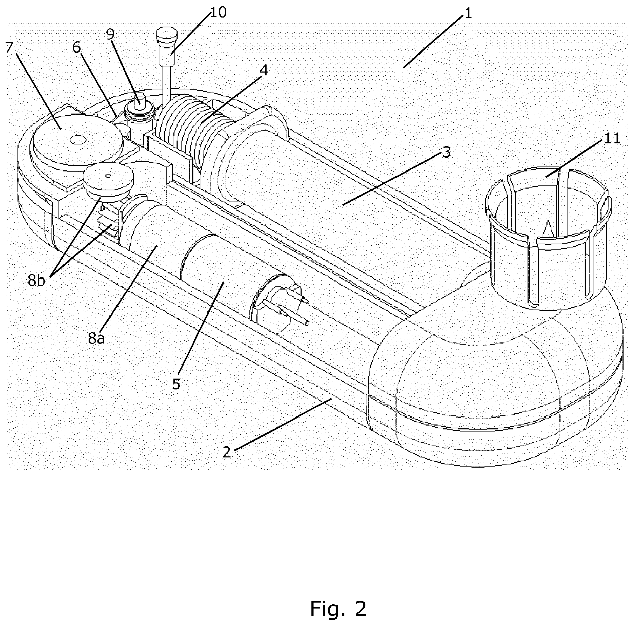

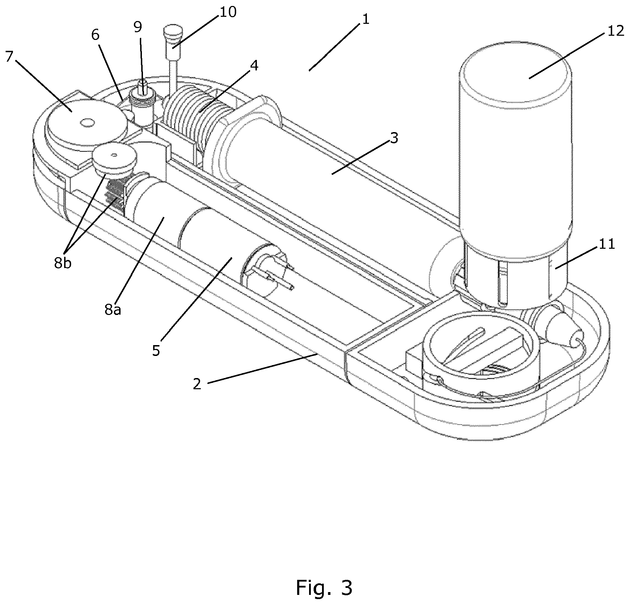

Figs. 1-3 are perspective views of an injection device 1 according to an embodiment of the invention. The injection device 1 comprises a housing 2 defining an interior part in which various parts of the injection device 1 are accommodated. In Figs. 2 and 3 part of the housing 2 has been removed in order to reveal the parts arranged in the interior part.

A syringe 3 with a movable plunger (not visible) is arranged inside the housing 2. It should be noted that it is not ruled out that the syringe 3 is provided with two or more movable plungers. This could, e.g., be the case for dual-chamber syringes. Furthermore, a driver mechanism is arranged inside the housing 2, the driver mechanism comprising a compressible spring 4 arranged in contact with the plunger in order to move the plunger in a first direction, and a motor 5 connected to a cable 6 which in turn is connected to the plunger in order to move the plunger in a second direction by pulling the cable 6 by means of the motor 5.

The motor 5 is connected to a winding mechanism 7, onto which the cable 6 is wound, via a gear mechanism 8, including a gear box 8a and connecting gear wheels 8b. The cable 6 is connected between the winding mechanism 7 and the plunger via a number of pulleys 9, one of which is visible. Thereby the cable 6 changes direction, and this provides a compact design of the injection device 1.

The injection device 1 further comprises a locking mechanism comprising a movable locking pin 10. When the locking pin 10 is arranged in a locked position, it locks the compressible spring 4, thereby preventing the compressible spring 4 from expanding and release mechanical energy stored therein. Accordingly, the spring 4 is prevented from moving the plunger in the first direction. When the locking pin 10 is moved to an unlocked position, it no longer locks the spring 4. Thereby the spring 4 is allowed to expand, releasing the mechanical energy stored therein, and moving the plunger in the first direction.

Furthermore, the injection device 1 comprises a vial adapter 11 arranged to receive a vial 12 (shown in Fig. 3), e.g. containing a medical drug. For instance, the vial 12 may contain a medical drug in a liquid form, which can be directly retrieved into the syringe 3. Alternatively, the vial 12 may contain a medical drug in a solid form, e.g. a lyophilized medical drug or a powder, in which case a diluent must be applied to the vial 12, and reconstitution of the drug must be performed before the medical drug can be retrieved into the syringe 3. Such diluent may be contained in the syringe 3. The vial 12 may contain only a single dose of medical drug, or it may contain multiple doses. In the latter case, the vial 12 may be arranged in the vial adapter 11 of another injection device 1 at a later point in time.

The injection device 1 of Figs. 1-3 may be operated in the following manner. During storage the locking pin 10 is in the locked position, thereby locking the spring 4 and preventing the spring 4 from moving the movable plunger. When it is desired to operate the injection device 1, a vial 12 is mounted in the vial adapter 11, and the locking pin 10 is moved to the unlocked position in order to unlock the spring 4. Thereby the spring 4 is allowed to expand and push the plunger in the first direction, thereby causing diluent to be transferred from the syringe 3 to the vial 12 in order to reconstitute or dilute a medical drug contained in the vial 12. Then the motor 5 is operated to pull the cable 6 in such a manner that the plunger of the syringe 3 is moved in the second direction, i.e. in a direction towards the proximal end of the syringe 3. Thereby medical drug is retrieved from the vial 12 into the syringe 3. Simultaneously, the compressible spring 4 is compressed by means of the cable 6, and thereby mechanical energy is stored in the spring 4. The plunger may be held in this position by means of the cable 6. Alternatively or additionally, the locking pin 10 may be moved to the locking position.

Next an injection needle is mounted on the syringe 3 and inserted at an injection position. The spring 4 is then allowed to expand and push the plunger in the first direction, i.e. in a direction towards the distal end of the syringe 3. This causes medical drug to be expelled from the syringe 3 via the injection needle.

During release of the mechanical energy stored in the spring 4, the movement of the plunger is controlled by controlling unwinding of the cable 6 from the winding mechanism 7. Accordingly, the position of the plunger as well as the speed of the movement of the plunger in the first direction is controlled accurately, and an accurate delivery of the medical drug is thereby obtained.

It should be noted that, in the case that the medical drug contained in the vial 12 is in a liquid form, the step of transferring a diluent from the syringe 3 to the vial 12 can be omitted and the liquid drug can simply be retrieved into the syringe 3 directly from the vial 12.

The injection device 1 of Figs. 1-3 may comprise a reusable sub-system and a disposable sub-system being assembled to form the injection device 1. In this case the reusable sub-system may comprise relatively expensive parts which are suitable for reuse, such as the motor 5, a battery, electronics, connectivity interfaces, sensors, displays, etc. The disposable sub-system may comprise parts which are brought into contact with the medical drug during use, and/or which require sterile conditions, e.g. the syringe 3 and possibly part of the driver mechanism.

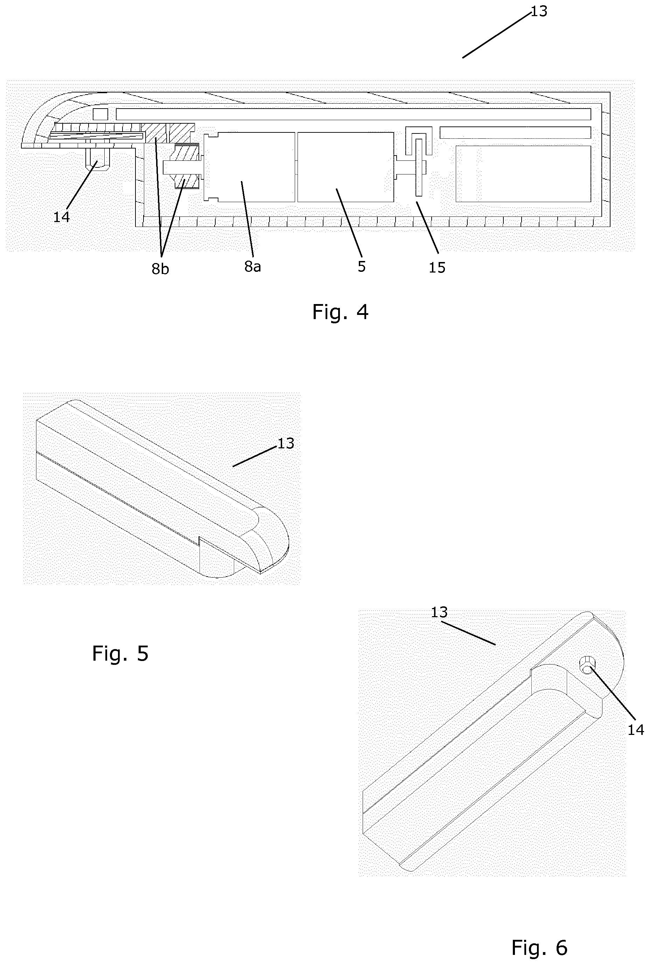

Figs. 4-6 illustrate a reusable sub-system 13 for an injection device according to an embodiment of the invention, e.g. the injection device 1 of Figs. 1-3. Fig. 4 is a cross sectional view of the reusable sub-system 13 and Figs. 5 and 6 are perspective views of the reusable sub-system 13 from two different angles.

In Fig. 4 it can be seen that the reusable sub-system 13 accommodates the motor 5. The motor 5 is coupled to a spline shaft 14 via a gear mechanism 8, including a gearbox 8a and connecting gear wheels 8b. Operating the motor 5 thereby causes the spline shaft 14 to rotate. The spline shaft 14 is arranged to engage a winding mechanism accommodated in a disposable sub-system, and thereby the rotating movements of the spline shaft 14 can be transferred to the winding mechanism and cause the cable to be wound or unwound. An optical rotation sensor 15 monitors rotational movements of the motor 5, thereby allowing the movements of the motor 5, and thereby of the cable, to be tracked.

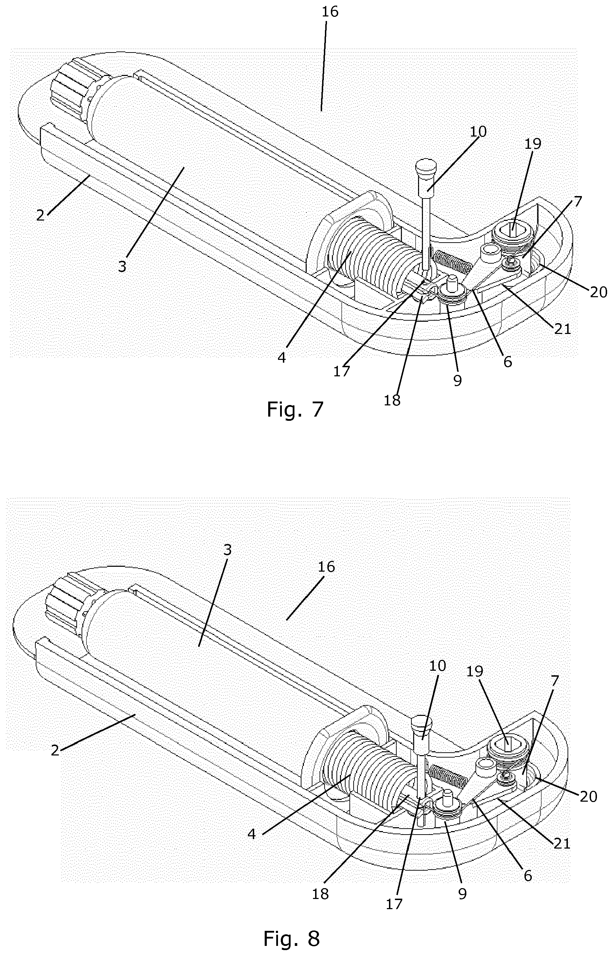

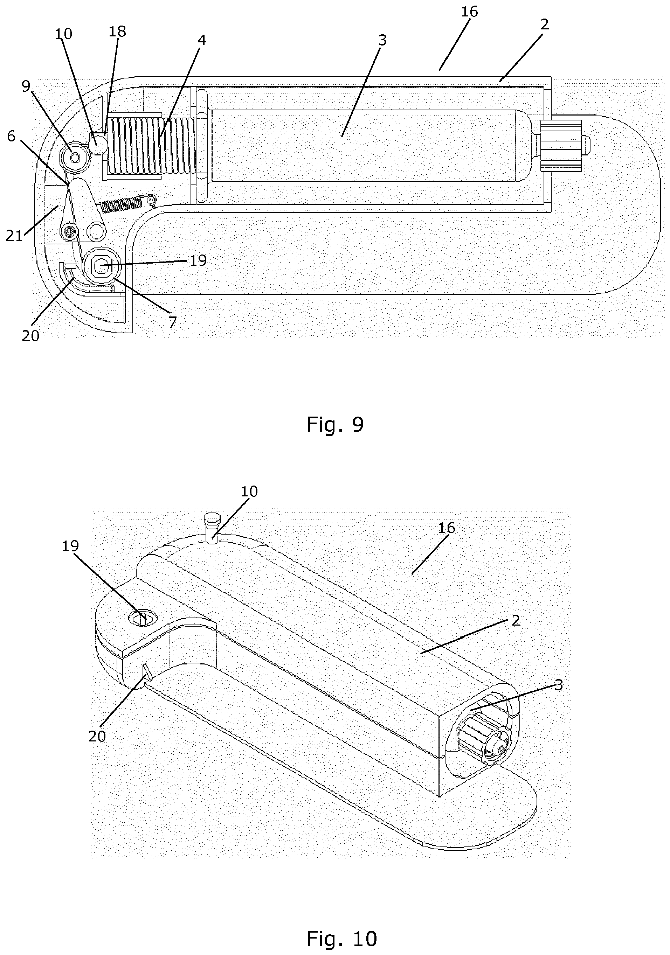

Figs. 7-10 illustrate a disposable sub-system 16 for an injection device according to an embodiment of the invention, e.g. the injection device 1 of Figs. 1-3. Figs. 7, 8 and 10 are perspective views of the disposable sub-system 16, and in Figs. 7 and 8 a part of the housing 2 has been removed in order to reveal parts accommodated inside the disposable sub-system 16. Fig. 9 is a top view of the disposable sub-system 16.

The disposable sub-system 16 accommodates the syringe 3 and part of the driver mechanism, including the compressible spring 4, the cable 6, the winding mechanism 7 and the pulley 9. Furthermore, the locking pin 10 is shown. In Fig. 7 the locking pin 10 is in an unlocked position in which it is not engaging an opening 17 formed in a movable member 18 which is arranged in contact with the movable plunger inside the syringe 3. Thereby the movable member 18 is allowed to move due to the spring force of the spring 4 acting thereon, the movable plunger being moved along.

In Fig. 8 the locking pin 10 is engaging the opening 17, and thereby the movable member 18 is prevented from moving and the spring 4 is prevented from expanding.

The locking pin 10 is normally in the locked position during storage of the disposable sub-system 16, in order to prevent that the movable plunger moves unintentionally during storage. This also serves as an indication regarding whether or not a disposable sub-system 16 has been used or compromised. If the locking pin 10 is in the locked position, this is an indication that the disposable sub-system 16 has not been used or compromised, and that it is therefore safe to use the disposable sub-system 16. On the other hand, if the locking pin 10 is in the unlocked position, this is an indication that the spring 4 has previously been released, and that the plunger has therefore been allowed to move in the first direction. Accordingly, the disposable sub-system 16 has either been used or compromised, and it is therefore not safe to use the disposable sub-system 16, and the disposable sub-system 16 should therefore be discarded.

The winding mechanism 7 comprises a female spline connector 19 arranged to receive a spline shaft of a reusable sub-system in order to transfer movements caused by operation of a motor accommodated in a reusable sub-system to the winding mechanism 7.

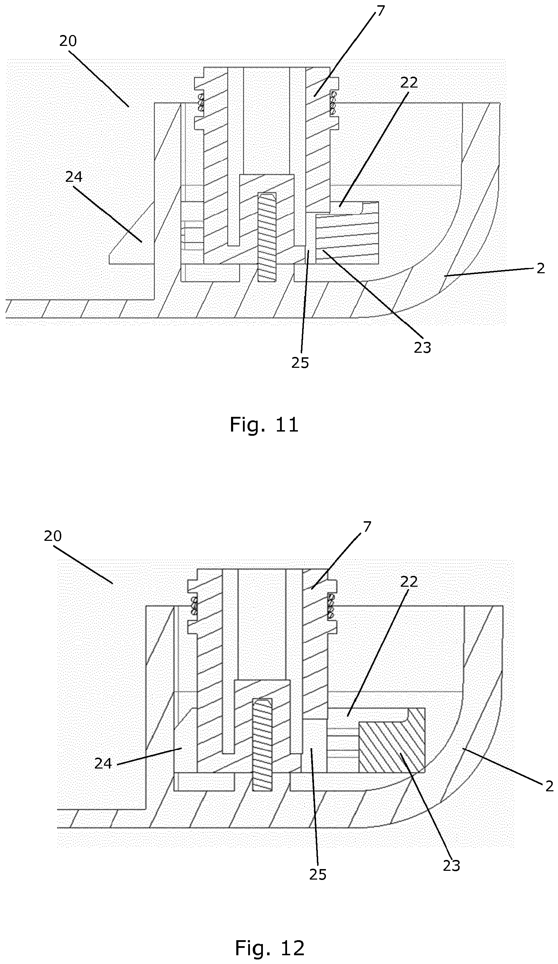

A further locking mechanism 20 is arranged at the winding mechanism 7. This locking mechanism 20 may be brought into a locked position in which it prevents the winding mechanism 7 from rotating, thereby preventing the cable 6 from being wound or unwound. Thus, when the further locking mechanism 20 is in the locked position, the plunger is prevented from moving. The further locking mechanism 20 will be described in further detail below with reference to Figs. 11 and 12.

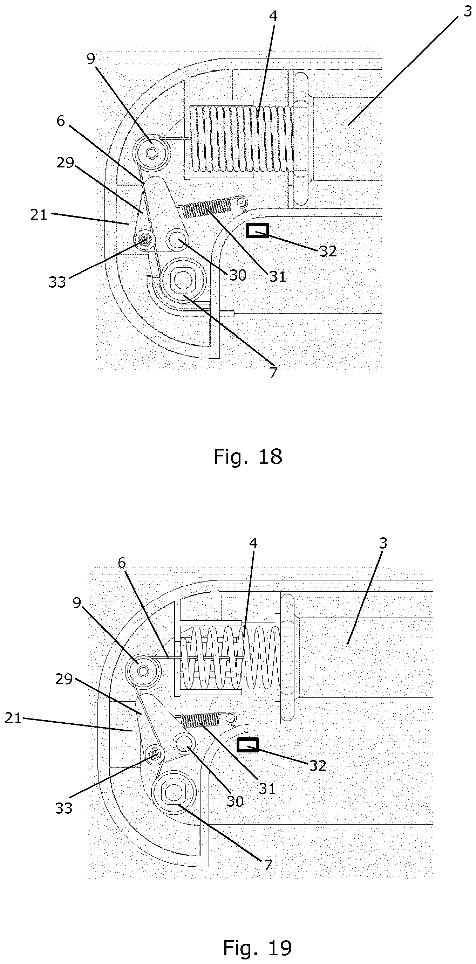

The disposable sub-system 16 further accommodates an arrangement 21 for detecting occlusion of the syringe 3, i.e. for detecting that the plunger is not moving as expected, e.g. due to drug clogging, plunger stiction, a bent needle, etc., or due the plunger having reached the end of the syringe 3. The arrangement 21 will be described in further detail below with reference to Figs. 18 and 19.

Figs. 11 and 12 illustrate the further locking mechanism 20 of the disposable sub-system 16 of Figs. 7-10 in further detail. Fig. 11 shows the locking mechanism 20 in a locked position and Fig. 12 shows the locking mechanism 20 in an unlocked position.

A slidable member 22 comprising an engagement portion 23 and a protruding portion 24 is mounted at the winding mechanism 7. The slidable member 22 is arranged to move between a position in which the engagement portion 23 is arranged in engagement with a groove 25 formed in the winding mechanism 7 and a position in which the engagement portion 23 is arranged out of engagement with the groove 25. When the engagement portion 23 is arranged in engagement with the groove 25, it prevents the winding mechanism 7 from rotating, and thereby the cable is prevented from being wound or unwound. Accordingly, the movable plunger of the syringe is prevented from moving, and therefore the locking mechanism 20 is in a locked position. This is the situation illustrated in Fig. 11.

When the engagement portion 23 is arranged out of engagement with the groove 25, the winding mechanism 7 is no longer prevented from rotating, and thereby the cable can be wound or unwound, subject to operation of a motor and/or subject to a spring force acting on the movable plunger. Accordingly, the movable plunger of the syringe is allowed to move, and therefore the locking mechanism is in an unlocked position. This is the situation illustrated in Fig. 12.

When the locking mechanism 20 is in the locked position, as illustrated in Fig. 11, the protruding portion 24 protrudes from the housing 2. Thereby the slidable member 22 can be moved from the locked position to the unlocked position by pushing the protruding portion 24 into the housing 2, i.e. to the position shown in Fig. 12. When a disposable sub-system accommodating the winding mechanism 7 and the locking mechanism 20 is assembled with a reusable sub-system, the protruding portion 24 may be automatically pushed into the housing 2 by the reusable sub-system. Thereby the locking mechanism 20 can be in the locked position during storage of the disposable sub-system, while it is ensured that the locking mechanism 20 is moved to the unlocked position when the disposable sub-system is assembled with a reusable sub-system. Accordingly, it is ensured that the plunger is not accidentally moved during storage, and that the plunger is allowed to move when it is desired to deliver the medical drug from the syringe.

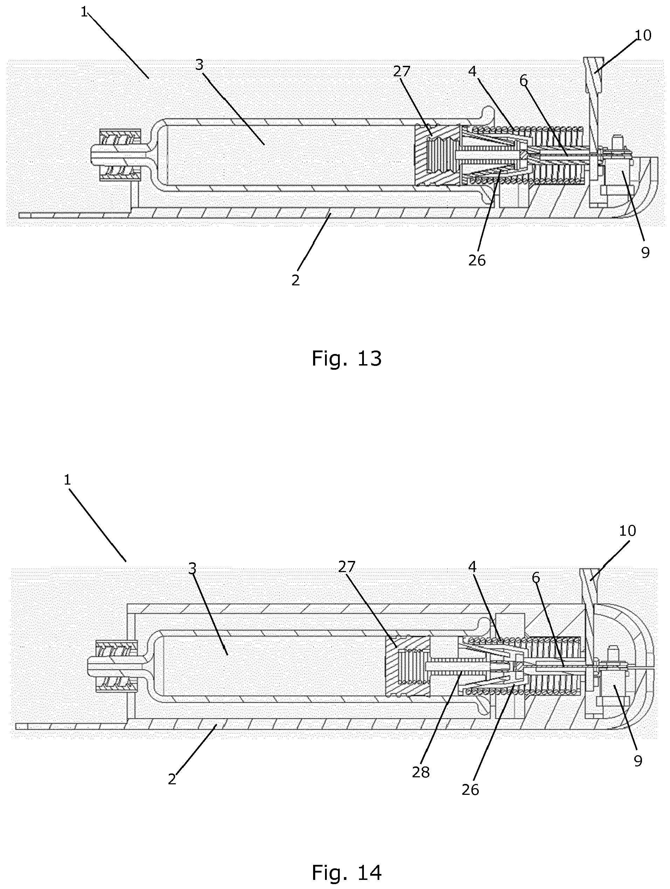

Fig. 13 is a cross sectional view of an injection device 1 according to an embodiment of the invention, comprising a pre-mounted syringe 3. The cable 6 extends through the centre of the spring 4 and is attached to a connecting arrangement 26 which is permanently attached to the movable plunger 27 arranged inside the syringe 3.

Fig. 14 is a cross sectional view of an injection device 1 according to an embodiment of the invention, comprising a user inserted syringe 3. In the embodiment of Fig. 14, the cable 6 is attached to a connecting arrangement 26 which is in turn connected to the plunger 27 via an adapter 28. Accordingly, when the user inserts the syringe 3 in the injection device 1, the adapter 28 clicks onto the connecting arrangement 26, thereby attaching the cable 6 to the plunger 27.

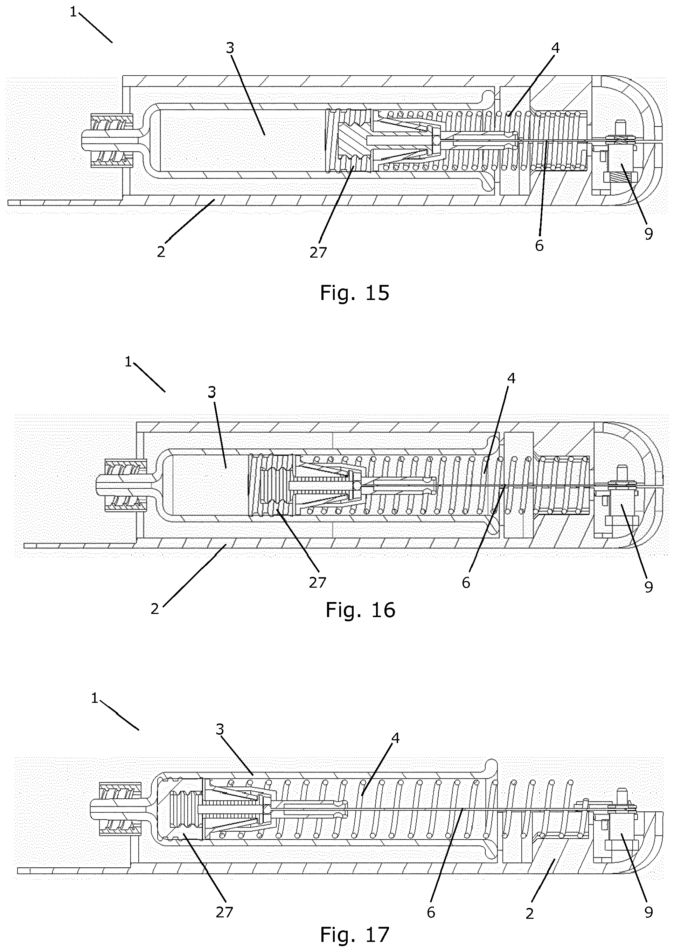

Figs. 15-17 illustrate operation of an injection device 1 according to an embodiment of the invention. In Fig. 15 the mechanical energy stored in the spring 4 has recently been released, and therefore the plunger 27 has started moving in a direction towards the distal end of the syringe 3, due to the spring force of the spring 4 acting on the plunger 27. It can be seen that the spring 4 has started expanding. In Fig. 15, the plunger 27 has been moved approximately one third of the distance along the syringe 3, and accordingly approximately one third of the medical drug contained in the syringe 3 has thereby been expelled.

In Fig. 16 the plunger 27 has moved further in the direction towards the distal end of the syringe 3. The plunger 27 has been moved approximately two thirds of the distance along the syringe 3, and accordingly approximately two thirds of the medical drug contained in the syringe 3 has thereby been expelled.

In Fig. 17 the plunger 27 has reached the end of the syringe 3, and thereby all of the medical drug contained in the syringe 3 has been expelled, i.e. the syringe 3 is now empty.

During the movement of the plunger 27 illustrated in Figs. 15-17 and described above, the cable 6 is kept tight, and the cable 6 thereby restricts the movement of the plunger 27 caused by the spring force of the spring 4. This allows the movement of the plunger 27, and thereby the speed of injection of the medical drug, to be accurately controlled.

Figs. 18 and 19 show an occlusion detection mechanism 21 for an injection device according to an embodiment of the invention. The occlusion detection mechanism 21 comprises a pivotally mounted element 29 having a magnet 30 mounted thereon. The pivotally mounted element 29 is biased towards the position shown in Fig. 19 by means of a spring 31. A magnetic Hall sensor 32 is fixedly mounted in the injection device and is used for detecting the proximity of the magnet 30 to the magnetic Hall sensor 32. Thereby the position of the pivotally mounted element 29 can be detected by means of the magnetic Hall sensor 32.

The occlusion detection mechanism 21 can be used for detecting occlusion in the following manner. When the movable plunger moves in an expected manner, for instance correctly following the movements of the motor, the cable 6 is kept tight. Thereby the cable 6 pulls the pivotally mounted element 29, via pulley 33, and moves the pivotally mounted element 29 against the spring force provided by spring 31. This is the situation illustrated in Fig. 18.

However, if the plunger stops or moves at a slower speed than expected, then there will no longer be a correspondence between the cable 6 being unwound from the winding mechanism 7 and the actual movement of the plunger. As a consequence, the cable 6 is no longer kept tight and is no longer capable of holding the pivotally mounted element 29 in the position shown in Fig. 18. Therefore the spring 31 pulls the pivotally mounted element 29 into the position shown in Fig. 19, bringing the magnet 30 closer to the magnetic Hall sensor 32.

Thus, when the plunger stops or moves at a slower speed than expected, e.g. due to occlusion or because the end of the syringe 3 has been reached, this can be detected by means of the magnetic Hall sensor 32.

## Claims

1. An injection device comprising:
- a housing,
- a syringe arranged inside the housing, the syringe having a movable plunger arranged therein,
- a driver mechanism arranged inside the housing for driving the movable plunger of the syringe,
wherein the driver mechanism comprises a spring and a cable operatively connected to the movable plunger of the syringe in such a manner that the movable plunger can be moved in a first direction by means of a spring force applied by the spring, and the movable plunger can be moved in a second direction being opposite to the first direction by means of a pulling force applied via the cable.

2. An injection device according to claim 1, wherein the spring is a compressible spring, and wherein movement of the movable plunger in the second direction by means of the pulling force applied via the cable further causes the spring to be compressed.

3. An injection device according to claim 1 or 2, wherein the cable is further arranged to control a velocity of movement of the plunger when the plunger is moved in the first direction by means of the spring force.

4. An injection device according to any of the preceding claims, wherein the driver mechanism further comprises a motor arranged to provide the pulling force via the cable.

5. An injection device according to claim 4, further comprising a coupling mechanism for coupling the motor and the cable.

6. An injection device according to claim 5, wherein the coupling mechanism comprises a gear mechanism.

7. An injection device according to claim 5 or 6, wherein the coupling mechanism comprises a spline connection.

8. An injection device according to any of the preceding claims, wherein the driver mechanism further comprises one or more pulleys arranged to guide the cable.

9. An injection device according to any of the preceding claims, further comprising position monitoring means for monitoring a position of the movable plunger.

10. An injection device according to claim 9, wherein the position monitoring means comprises an optical sensor.

11. An injection device according to claim 9, wherein the position monitoring means comprises a movably mounted magnet and a magnetic Hall sensor configured to detect a proximity of the movably mounted magnet.

12. An injection device according to claim 9, wherein the position monitoring means forms part of a motor arranged to provide the pulling force via the cable.

13. An injection device according to claim 12, wherein the position monitoring means is arranged to monitor motor input current of the motor.

14. An injection device according to any of the preceding claims, further comprising a locking mechanism being configured to be in a locked position in which the spring is prevented from applying a spring force to the movable plunger and in an unlocked position in which the spring is allowed to apply a spring force to the movable plunger.

15. An injection device according to any of the preceding claims, wherein the driver mechanism forms part of a disposable sub-system being removable from the housing.
